# EUROPÄISCHE PATENTSCHRIFT

(11) **EP 0 537 427 B1**
(45) Veröffentlichungstag und Bekanntmachung des Hinweises auf die Patenterteilung: **24.01.1996**
(21) Anmeldenummer: 92113052.2
(22) Anmeldetag: 31.07.1992
(51) Int. Cl.: C08H 5/00

(54) **Verfahren zur Herstellung von Huminaten**
Process for producing huminates
Procédé de production d'huminates

(30) Priorität: 17.10.1991 DE 4134379
(43) Veröffentlichungstag der Anmeldung: 21.04.1993
(73) Patentinhaber: RÜTGERSWERKE AKTIENGESELLSCHAFT, D-60326 Frankfurt (DE)
(72) Erfinder: Seubert, Bernhard, W-6803 Edingen (DE); Riede, Urs. N., Prof. Dr., W-7800 Freiburg (DE)

(56) Entgegenhaltungen:
- EP-A- 0 281 678
- CHIMIKA CHRONIKA Bd. 1, 1972, GR Seiten 203 - 209 S. PARASKEWAS 'über die Autoxydation von Hydrochinon in Alkalischen Losungen Mittels Luftsauerstoff'

## Beschreibung

Die Erfindung betrifft die synthetische Herstellung von Ammonium- oder Alkalisalzen physiologisch wirksamer, nur gering toxischer und nicht mutagener Huminsäuren.

Aus DE-A 37 07 909 ist bekannt, daß den Alkalisalzen niedermolekularer Huminsäuren eine Heilwirkung zukommt, und die Huminate, im Gegensatz zu bis dahin bekannten Huminstoffen, bzw. deren Salzen, wesentlich weniger toxisch sind.

Diese niedermolekularen Huminstoffe können entweder aus natürlich vorkommenden Huminstoffen isoliert oder synthetisch hergestellt werden, wie dies aus DE-A 37 07 910 bekannt ist. Dabei werden mehrwertige Phenole in alkalischer Lösung oxidiert, wobei der pH-Wert der Reaktionsmischung im Bereich von 8,8 bis 9,0 liegt. Durch diese Reaktionsbedingung ist gewährleistet, daß nur niedermolekulare Huminate entstehen, deren mittleres Molekulargewicht bei 1000 liegt, bei einem Streubereich von 300 bis 1500.

Lediglich diese Huminate werden als physiologisch wirksam aber als nicht toxisch, nicht mutagen und nicht teratogen angesehen.

Die Einstellung und Einhaltung eines pH-Wertes im engen Bereich von pH 8,8 bis 9,0 und der bei der Aufarbeitung einzustellende pH-Wert im Bereich von 6,2 bis 7,2 sind für technische Prozesse insofern ungünstig, als die Pufferkapazität von wäßrigen Lösungen im Bereich von pH 6,0 bis 9,0 äußerst gering ist, so daß durch kleine Änderungen der Konzentrationen von Säure oder Lauge pH-Werte des Reaktionsmilieus auftreten, die außerhalb des vorgeschriebenen Bereiches liegen.

Es ist daher Aufgabe der Erfindung, ein technisch einfach zu beherrschendes Verfahren zur synthetischen Herstellung physiologisch wirksamer, aber nur gering toxischer und nicht mutagener Huminate bereitzustellen. Eine weitere Aufgabe ist die Erweiterung der Palette der physiologisch wirksamen Huminate, die als Heilmittel eingesetzt werden können.

Die Lösung der Aufgabe erfolgt durch ein Verfahren zur Herstellung der Huminate gemäß der Ansprüche 1 bis 3, die so hergestellten Huminate nach Anspruch 4 und ihre Verwendung nach den Ansprüchen 5 bis 11.

Entgegen der Erkenntnisse früherer Anmeldungen wurde gefunden, daß für die gewünschten Eigenschaften der physiologischen Wirksamkeit im Sinne eines Heilmittels bei gleichzeitiger geringer Toxizität und Nicht-Mutagenität, bzw. -Teratogenität von Huminstoffen nicht deren Molekulargewicht im Bereich von 300 bis 1500 ausschlaggebend ist, sondern daß die Auswahl durch andere Kriterien bestimmt wird. Zwar erfüllen die oben genannten niedermolekularen Huminate mit dem mittleren Molekulargewicht von 1000, bei einem Streubereich von 300 bis 1500 die entsprechenden Bedingungen.

Darüber hinaus gibt es aber weitere Huminate mit höherem Molekulargewicht (bis etwa 50 000 D), die ebenfalls die bekannten heilenden und erwünschten physiologischen Eigenschaften besitzen, die aber nur sehr gering toxisch und die weder mutagen noch teratogen sind.

Die gemeinsamen Merkmale dieser positiv physiologisch wirkenden Huminate sind, daß ihre wäßrigen Lösungen keinen Tyndall-Effekt zeigen und nicht fluoreszieren. Entsprechende Prüfungen dieser Eigenschaften werden zweckmäßigerweise mit wäßrigen Lösungen in einer Konzentration durchgeführt, daß sie noch etwa 50 % der Transmission des eingestrahlten Lichtes haben.

Es wurde gefunden, daß sich diese Huminate in einfacher Weise durch Oxidation mehrwertiger Phenole in alkalischem, wäßrigem Medium bei einer Reaktionstemperatur unter 40 °C herstellen lassen, wenn der pH-Wert des Reaktionsmediums während der Oxidation immer über 9,0 liegt und die Zuführung des Oxidationsmittels so dosiert wird, daß der Gehalt der den mehrwertigen Phenolen entsprechenden Chinonen immer kleiner als 0,5 % ist, bezogen auf die eingesetzten mehrwertigen Phenole, und daß die Oxidationsreaktion abgebrochen wird, wenn die Chinonkonzentration trotz Zufuhr von Oxidationsmittel absinkt.

Bevorzugt wird die Oxidationsreaktion abgebrochen, wenn die Chinonkonzentration trotz Zufuhr von Oxidationsmittel unter 0,05 %, bezogen auf die eingesetzten mehrwertigen Phenole, abgesunken ist.

Ausgangsprodukte für das erfindungsgemäße Verfahren können alle gängigen mehrwertigen Phenole sein, wie Brenzkatechin, Resorcin, Hydrochinon, Orcin, Gallussäure, Protocatechusäure, Pyrogallol, 2-Oxyhydrochinon, Phloroglucin oder Tetraoxybenzole. Die mehrwertigen Phenole können als Reinsubstanzen oder im beliebigen Gemisch miteinander eingesetzt werden. Das bevorzugte mehrwertige Phenol ist Hydrochinon.

Zur Umsetzung werden die mehrwertigen Phenole in einer alkalischen, wäßrigen Lösung gelöst. Als Laugen können alle Alkalimetallhydroxide, Ammoniak oder starke Amine in wäßriger Lösung verwendet werden. Aus wirtschaftlichen Gründen sind Natrium und Kaliumhydroxid bevorzugt. Entscheidend für die erfindungsgemäße Reaktion ist die Menge des eingesetzten Alkalihydroxids. Sie muß oberhalb der 1,6 -fachen stöchiometrischen Menge liegen, die zur Phenolatbildung der mehrwertigen Phenole, das heißt, zur Neutralisation aller phenolischen OH-Gruppen notwendig ist. Dabei stellt sich ein pH-Wert der Reaktionslösung ein, der oberhalb von pH 9,0 liegt.

Die Alkalität der Reaktionslösung kann zum alkalischen Bereich hin unbegrenzt sein. Da aber das Reaktionsgemisch zur Aufarbeitung neutralisiert werden muß, ist es aus ökonomischen Gründen angebracht, die Lösung nicht zu stark alkalisch zu machen, sondern zu versuchen, im Grenzbereich oberhalb pH 9,0 zu liegen und ein Absinken des pH-Wertes während der Reaktion gegebenenfalls durch nachträgliche Zugaben von Lauge zu verhindern.

Es ist einleuchtend, daß die eingesetzten Verbindungen möglichst rein sein sollen, denn dadurch werden unerwünschte Nebenreaktionen vermieden, bzw. die erhaltenen Endprodukte lassen sich ohne weitere chemische Aufarbeitung - die auch mit unerwünschten chemischen Veränderungen der Endprodukte verbunden wären - erhalten und im erhaltenen Reaktionsmedium einsetzen.

Im allgemeinen ist es ausreichend, wenn als Wasser ein entmineralisiertes Wasser mit einer Leitfähigkeit von 6 bis 10 µS/cm und einem pH-Wert im Bereich von 5 bis 7 eingesetzt wird und als Alkalihydroxid entweder die Qualität "chemisch rein" oder der Reinheitsgrad nach DAB 9.

Als mehrwertiges Phenol sollte ebenfalls eine "chemisch reine" Qualität eingesetzt werden mit einem Gehalt an mehrwertigem Phenol von mehr als 98 %.

Die Oxidation des mehrwertigen Phenolats kann entweder elektrochemisch oder plasmachemisch, bzw. chemisch durch Durch- oder Überleiten von Sauerstoff oder eines sauerstoffhaltigen Gasgemisches erfolgen.

Die elektrochemische Oxidation erfolgt in einem elektrochemischen Reaktor, wobei die Oxidationsgeschwindigkeit durch Einstellung der Anodenspannung und der Stromdichte bestimmt wird.

Die Anodenspannung kann im Bereich von 4 bis 15 V und die Stromdichte im Bereich von 0,5 bis 4 A/cm variiert werden. Dabei liegt die Reaktionszeit im Bereich von 1 bis 3 d.

Die plasmachemische Oxidation erfolgt in einer an sich bekannten Apparatur zur Coronaentladung, wobei die Oxidationsgeschwindigkeit durch Einstellung der Betriebsspannung und der Feldstärke bestimmt wird. Die Spannung kann im Bereich von 20 bis 250 kV bei Frequenzen von 16 2/3 bis 400 Hz und die Feldstärke von 80 KV/cm bis 200 KV/cm variiert werden. Dabei liegt die Reaktionszeit im Bereich von 15 bis 120 Minunten.

Zur chemischen Oxidation wird die alkalische Lösung des mehrwertigen Phenolats in ein Reaktionsgefäß gebracht, das den unkontrollierten Zutritt von Luft verhindert, das heißt, in ein geschlossenes Reaktionsgefäß, das aber mit einer Vorrichtung versehen ist, mittels der Gase ein- bzw. durchgeleitet werden können.

Nun wird unter Rühren Sauerstoff oder ein sauerstoffhaltiges Gasgemisch in ständigem Gasstrom entweder über oder durch die Reaktionslösung oder unter Druck auf die Reaktionslösung geleitet, wobei die Temperatur des Reaktionsgemisches im Bereich von 10 bis 40 °C, bevorzugt im Bereich von Raumtemperatur bis 30 °C liegt.

Als sauerstoffhaltiges Gasgemisch kann auch Luft eingesetzt werden, die aber vorher zu Adsorption von CO₂ und zur Entfernung von Staubpartikeln über einen alkalischen Filter geleitet werden muß.

Die chemische Oxidation kann auch durch Reaktion mit milden Oxidationsmitteln wie Wasserstoffperoxid, seinen Anlagerungsverbindungen oder Persulfaten erfolgen.

Während der Oxidationsreaktion werden der pH-Wert des Reaktionsgemisches und der Gehalt des Reaktionsgemisches an dem mehrwertigen Phenol entsprechenden Chinon überwacht. Sobald einer der beiden Parameter unter- bzw. überschritten wird, ist der Reaktionsansatz zu verwerfen.

Die Oxidationsreaktion wird über die Menge des eingebrachten Oxidationsmittels gesteuert, wobei der Begriff Oxidationsmittel je nach angewendetem Verfahren als chemisches Reagenz oder als angelegte Spannung und Stromstärke aufzufassen ist.

Die Reaktionsdauer beträgt in der Regel 10 bis 15 Tage. Die Reaktion wird beendet, wenn trotz weiterer Zugabe von Oxidationsmitteln der Gehalt an Chinon absinkt, bevorzugt dann, wenn die Chinonkonzentration auf einen Wert unter 0,05 %, bezogen auf die eingesetzten mehrwertigen Phenole, abgesunken ist.

Danach wird das Reaktionsgemisch in an sich bekannter Weise auf einen pH-Wert im Bereich von 4 bis 5 angesäuert und mittels an sich bekannter Methoden, wie z. B. Ultra -zentrifugation, -filtration oder Elektrophorese aufgearbeitet. So wird z. B. die aus der chemischen, plasmachemischen oder der elektrochemischen Oxidationsreaktion resultierende dunkelbraune Lösung auf einen pH-Wert im Bereich von 4 bis 5, bevorzugt von 4,5 eingestellt und gegebenenfalls gepuffert.

Dies erfolgt entweder durch Zugabe von Säure oder durch Einwirkung von saurem Ionenaustauscher und/oder nachfolgender Zugabe einer entsprechenden Pufferlösung.

Sofern die neutralisierte und gepufferte Lösung unerwünschte Schwebstoffe enthält, wird sie nun durch ein Trennverfahren wie Zentrifugieren (10 000 bis 30 000xg) oder Filtrieren durch ein sehr feinporiges Filtermaterial von diesen Schwebestoffen befreit. Obwohl diese Lösung für viele Anwendungszwecke direkt eingesetzt werden kann, sollte sie bei Verwendung der Produkte als Heilmittel durch an sich bekannte Reinigungsverfahren wie präparative Chromatographiemethoden, Ultrafiltration, Ultrazentrifugation oder Elektrodialyse gereinigt und von unerwünschten Nebenprodukten befreit werden.

Danach kann die erhaltene braune Lösung mit 3 bis 5 % Huminat aufkonzentriert (bis etwa 40 % Huminat) oder mit Hilfe eines geeigneten Trockners bis zu einer Restfeuchte von 2 % getrocknet werden. Bei der Trocknung wird eine leicht zu zerkleinernde, kristalline, hygroskopische Masse von schwarz glänzender Farbe erhalten.

Die verdünnte sowie die aufkonzentrierte Lösung und auch der erhaltene Feststoff sind stabil.

Bei Stabilitätskontrollen ist nach 60 Tagen Wechselbelastung 56/4 °C im 12/12 h Rhythmus keine Veränderung der Parameter Gehalt, pH-Wert, Redoxspannung und Mikrodialysetest über die Zufallsschwankungen hinaus feststellbar.

Toxizitätsprüfungen zeigen, daß die erhaltenen Huminate gering toxisch sind, andererseits wurde gefunden, daß die erfindungsgemäß hergestellten Huminate ebenfalls die bei den erwähnten niedermolekularen Huminstoffen beobachteten Heilwirkungen und detoxifizierenden Wirkungen zeigen.

Die erfindungsgemäßen Huminate eignen sich daher für alle Anwendungen, die von den natürlichen oder synthetischen niedermolekularen Huminstoffen bekannt sind.

Insbesondere eigenen sie sich zur Verwendung als Wundheilmittel, zur Herstellung von Mitteln zur Wundheilung, zur Herstellung von hochwirksamen Moorbädern, von Nasenarzneien gegen Pollenallergien, von Antischuppenshampoos sowie von Mitteln zur Behandlung von Fischen, insbesondere von gestreßten Fischen. Darüber hinaus eignen sie sich zur Detoxifizierung von Wasser sowie von mit chemischen Gefahrstoffen, insbesondere von mit cholinesterasehemmenden Stoffen vom Typ Organophosphate und/oder mit dermatotoxischen Stoffen vom Typ Dichlordiethylsulfid kontaminierten Flächen oder festen Gegenständen.

### BEISPIEL

In einem gegen Alkalien beständigen Rührwerkbehälter werden 95 kg Kaliumhydroxid (DAB 9-Qualität) in 1000 l entmineralisiertem Wasser (pH 5 bis 7; Leitfähigkeit unter 5 µS/cm) gelöst.

Nach dem Abkühlen auf eine Temperatur unterhalb 40 °C werden unter Kühlen im Verlauf von 45 Minuten 55 kg Hydrochinon (chem. rein., über 99 %) zugegeben, wobei die Temperatur des Gemisches 40 °C nicht übersteigt. Es stellt sich ein pH-Wert von etwa 10 ein.

Die Apparatur wird nun verschlossen. Dann wird unter Rühren Luft, die über ein alkalisches Filtersystem von Staub, CO₂ und anderen Kontaminanten gereinigt wird, über die Oberfläche geleitet.

Der Luftstrom wird so gedrosselt, daß der Gehalt an 1,4-Benzochinon immer kleiner ist als 0,5 %, bezogen auf das eingesetzte Hydrochinon. Die Reaktionstemperatur wird immer unter 40 °C gehalten. Der pH-Wert bleibt immer über 9,0.

Nach 10 bis 15 Tagen sinkt der Gehalt an Hydrochinon der braunen Reaktionslösung auf unter 2 %. Trotz weiterer Luftzufuhr sinkt der Gehalt an 1,4-Benzochinon auf Werte unter 0,05 %. In diesem Zeitpunkt wird die Reaktion abgebrochen. Die Luftzufuhr wird beendet und die Reaktionsmischung wird durch Zugabe eines sauren Ionenaustausches auf einen pH-Wert von 4,5 eingestellt. Danach wird der Ionenaustauscher durch zentrifugieren abgetrennt und die saure Lösung einer Ultrafiltration unterworfen, wobei ein Filtrationsgerät mit 0,5 µm Feinheit verwendet wird.

Das Ultrafiltrat wird unter schwachem Vakuum bis zu einer Huminat-Konzentration von 40 Gew.-% eingeengt. Diese Lösung ist noch gut handhabbar. Sie enthält Huminate mit Molekulargewichten im Bereich 1000 bis 50 000. Die Lösung zeigt keinen Tyndall-Effekt und enthält keine fluoreszierenden Komponenten.

### Toxität:

Bei Injektionen der 1 %igen Lösung an Versuchstieren (Mäuse) werden folgende Werte der LD 50 erhalten:
s.c. 1370 (mg/kg)
i.p. 920 (mg/kg)
i.v. 840 (mg/kg)

### Stabilität:

Nach einer 6 monatigen Lagerung unter Luftabschluß bei 23 °C sind keine Veränderungen feststellbar.

### Heilwirkung :

Eine nach Thrypsinbehandlung in Suspension gebrachte Kultur von L-Zellen (Mäusefibroblasten) wird mit 50 ppm Huminat versetzt.

Die Kultur wird bei 37 °C unter Verwendung eines handelsüblichen Nährmediums 48 Stunden lang bebrütet.

Parallel hierzu wird als Vergleichsversuch eine analoge Kultur ohne Huminate gleichermaßen bebrütet. Dananch wird die Anzahl der lebenden Zellen in beiden Kulturen bestimmt.

In der mit erfindungsgemäßem Huminat versetzten Kultur liegt die Zahl der lebenden Zellen um 30 % höher als in der Vergleichskultur.

### Wundheilung :

An 2 x 10 haarlosen Mäusen werden mit einem Mikrodermatom oberflächliche Wunden, die nur die obersten Epithelschichten erfaßten, in einer Größe von etwa 50 mm beigebracht.

Bei zehn dieser Mäuse wird die Wunde mit einer 1 %igen Huminat-Lösung einmal benetzt. Die anderen Mäuse bleiben unbehandelt. Während des Beobachtungszeitraumes von 7 d läßt sich folgendes Beobachten:

Gegenüber den unbehandelten Mäusen nimmt bei den behandelten Versuchstieren
die'Wundfläche rascher ab,
die Wunde trocknet früher ab,
die Granulation setzt früher ein und
die Wunde reinigt sich früher.

Insgesamt ist die Abheilung 2 bis 3 d früher als bei den Kontrolltieren zu beobachten.

### Wirkung bei gestreßten Fischen:

Jeweils 50 Fische verschiedener Arten wurden nach einem Transport in drei verschiedene Becken mit je 70 l Wasser eingebracht und im Verlauf von 7 d vergleichend beobachtet.

Die Haltungs- und Fütterungsbedingungen waren dabei jeweils gleich. Lediglich das im Prinzip gleiche Wasser war durch Zusätze verändert:
- Becken a:: kein Zusatz
- Becken b:: 20 ml (vorgeschriebene Dosis) eines handelsüblichen huminsäurehaltigen Prophylaktikums
- Becken c:: 3 ml einer 2 %igen wäßrigen Lösung des Huminats. Nach 4 d wurden weitere 2 ml der 2 %igen Lösung nachdosiert.

### Ergebnis:

Während in Becken a eine Mortalitätsrate von 18 % (Guppy), bzw. 50 % (roter Phantomsalmler) und eine generell schlechte Vitalität der Fische beobachtet wurde, war in Becken bdie Mortalität auf 10,5 % bzw. 19 % abgesunken und die Vitalität der überlebenden Fische war mäßig. In Becken c lag die Mortalität bei 5 % und die Vitalität war durchweg gut.

### Detoxifizierung von Wasser:

Geprüft wird die Toxität des gemäß Beispiel hergestellten Huminats gegenüber Daphnia magna (Prüfung nach OECD-Richtlinie).

Der Ausdruck LC 50 bezeichnet die Konzentration eines Stoffes in Wasser, die nach der angegebenen Zeit bei 50 % der getesteten Lebewesen letal wirkt.

LC 50 des erfindungsgemäßen Huminats: 2 700 mg/l

### Wirking bei schadstoffbelastetem Wasser:

| Schadstoff | Menge Schadstoff | Menge Huminat | Überlebensdauer [h] | LC 50 [mg/l] nach 6 h |
|---|---|---|---|---|
| Cadmium-chlorid | | 0 ppm | 0,2 | 0,5 |
| | 1 mg/l | 20 ppm | 3 | 2,5 |
| Bleiacetat | | 0 ppm | 0,5 | 2,5 |
| | 5 mg/l | 20 ppm | 6 | 7,5 |
| Quecksilber-1-chlorid | | 0 ppm | 0,5 | 0,5 |
| | 3 mg/l | 20 ppm | 1,5 | 2,8 |
| Parathion | | 0 ppm | 0,2 | 0,5 |
| | 4 mg/l | 20 ppm | 24 | 10,0 |
| Atrazin | | 0 ppm | 0,5 | 2,0 |
| | 4 mg/l | 20 ppm | 4,5 | 10,0 |

### Wirkung als Nasenarznei:

Eine Versuchsgruppe von 10 Personen, die unter durch Pollenflug bedingten allergischen Reaktionen, insbesondere im Nasen- und Augenbereich leiden, verwendeten eine 0,5 %ige wäßrige Lösung des bespielhaft hergestellten Huminates als Nasenspray, indem sie sich bei Auftreten der Heuschnupfensymptome mittels einer an sich bekannten und üblicherweise für Nasensprays verwendeten Vorratsflasche jeweils eine Dosis der Lösung in die Nase sprühten. Bei allen Patienten verringerten sich innerhalb von Minuten die Beschwerden. Die Nasen wurden frei, der Niesreiz verschwand, die Verquellung der Augen wurde reduziert. Unerwünschte Nebenwirkungen wurden auch nach längerem Gebrauch des Mittels nicht beobachtet. Hingegen berichteten sechs der Versuchspersonen, daß nach mehrtägigem Gebrauch des Mittels die Zahl der notwendigen Anwendungen sich wesentlich reduziert hat. An Tagen mit niedriger Pollenbelastung waren diese Patienten auch ohne das erfindungsgemäße Nasenspray beschwerdefrei.

### Wirkung des Antischuppenmittels:

Zu je 100 ml eines handelsüblichen Haarshampoos werden jeweils 50 ml einer 5 %igen wäßrigen Lösung des im Beispiel hergestellten Huminates zugegeben.

Verschiedene Probanden, die unter Kopfschuppen zu leiden hatten, verwandten jeweils eine der erhaltenen verdünnten Haarshampoos (jeweils 2 x 5 ml) bei der täglichen Wäsche von Haar und Kopfhaut.

Die Ergebnisse waren in allen Fällen gleich: Nach einer Anwendungszeit von etwa einer Woche war das Problem der Kopfschuppen nicht mehr akut. Auch nach mehrwöchigem Gebrauch der Shampoos war keine Erhöhung von Talgdrüsenaktivitäten auf der Kopfhaut zu beobachten. In einigen Fällen wurde subjektiv ein Rückgang der Haarfettung beobachtet.

## Patentansprüche

1. Verfahren zur Herstellung von physiologisch wirksamen, aber nicht toxischen, nicht teratogenen und nicht mutagenen Huminaten durch Oxidation mehrwertiger Phenole in alkalischem, wäßrigem Medium bei einer Reaktionstemperatur unter 40 °C und anschließende Isolierung, **dadurch gekennzeichnet,** daß der pH-Wert des Reaktionsmediums während der Oxidation immer über 9,0 liegt und die Zuführung des Oxidationsmittels so dosiert wird, daß der Gehalt der den mehrwertigen Phenolen entsprechenden Chinonen immer kleiner als 0,5 % ist, bezogen auf die eingesetzten mehrwertigen Phenole, und daß die Oxidationsreaktion abgebrochen wird, wenn die Chinonkonzentration trotz Zufuhr von Oxidationsmittel absinkt.

2. Verfahren nach Anspruch 1, **dadurch gekennzeichnet**, daß die Oxidationsreaktion abgebrochen wird, wenn die Chinonkonzentration trotz Zufuhr von Oxidationsmittel unter 0,05 %, bezogen auf die eingesetzten mehrwertigen Phenole, abgesunken ist.

3. Verfahren nach den Ansprüchen 1 und 2, **dadurch gekennzeichnet**, daß als mehrwertiges Phenol Hydrochinon verwendet wird.

4. Huminate, hergestellt nach einem Verfahren gemäß einem der Ansprüche 1 bis 3.

5. Verwendung der Huminate nach Anspruch 4, zur Herstellung von Mitteln zur Wundheilung.

6. Verwendung der Huminate, nach Anspruch 4, zur Herstellung von hochwirksamen synthetischen Moorbädern.

7. Verwendung der Huminate, nach Anspruch 4, zur Herstellung eines Mittels zur Behandlung gestreßter Fische.

8. Verwendung der Huminate, nach Anspruch 4, zur Detoxifizierung von Wasser.

9. Verwendung der Huminate, nach Anspruch 4, zur Detoxifizierung von Flächen und festen Gegenständen.

10. Verwendung der Huminate, nach Anspruch 4, zur Herstellung einer Nasenarznei.

11. Verwendung der Huminate, nach Anspruch 4, zur Herstellung eines Antischuppenmittels.

## Claims

1. A process for the manufacture of physiologically effective yet nontoxic, non-teratogenic and non-mutagenic huminates, by oxidation of polyvalent phenols in an alkaline aqueous medium at a reaction temperature below 40°C and subsequent isolation, **characterised in that** the pH value of the reaction medium is above 9.0 throughout oxidation, and the addition of the oxidation agent is metered in such a manner that the content of quinones corresponding to the polyvalent phenols is always less than 0.5% in relation to the polyvalent phenols used, and the oxidation reaction is halted when the quinone concentration falls despite addition of oxidation agents.

2. A process in accordance with claim 1, **characterised in that** the oxidation reaction is halted when, despite the addition of oxidation agents, the quinone concentration has fallen below 0.05% in relation to the polyvalent phenols used.

3. A process in accordance with claims 1 and 2, **characterised in that** hydroquinone is used as a polyvalent phenol.

4. Huminates, produced using a process in accordance with one of claims 1 to 3.

5. The use of huminates in accordance with claim 4, to produce wound-healing agents.

6. The use of huminates in accordance with claim 4, to produce highly effective synthetic mud baths.

7. The use of huminates in accordance with claim 4, to produce an agent for treating stressed fish.

8. The use of huminates in accordance with claim 4, to detoxify water.

9. The use of huminates in accordance with claim 4, to detoxify surfaces and solid objects.

10. The use of huminates in accordance with claim 4, to produce a nasal medicament.

11. The use of huminates in accordance with claim 4, to produce an antidandruff agent.

## Revendications

1. Procédé de préparation d'humates physiologiquement actifs, mais non toxiques, non tératogènes et non mutagènes par oxydation de polyphénols en milieu aqueux alcalin à une température de réaction inférieure à 40°C et isolement subséquent, caractérisé en ce que la valeur de pH du milieu réactionnel pendant l'oxydation se situe toujours au-dessus de 9,0 et que l'apport de l'oxydant est dosé de telle façon que la teneur en les quinones correspondant aux polyphénols soit toujours inférieure à 0,5%, rapporté aux polyphénols mis en oeuvre et que la réaction d'oxydation est interrompue lorsque la concentration de quinone diminue malgré l'apport d'oxydant.

2. Procédé selon la revendication 1, caractérisé en ce que la réaction d'oxydation est interrompue lorsque la concentration de quinone, malgré l'apport d'oxydant, est tombée au-dessous de 0,05%, rapporté aux polyphénols mis en oeuvre.

3. Procédé selon les revendications 1 et 2, caréactérisé en ce que de l'hydroquinone est utilisée en tant que polyphénol.

4. Humates préparés selon un procédé conforme à l'une des revendications 1 à 3.

5. Utilisation des humates,selon la revendication 4,pour la préparation d'agents pour la cicatrisation.

6. Utilisation des humates, selon la revendication 4, pour la préparation de bains de boue synthétiques hautement efficaces.

7. Utilisation des humates,selon la revendication 4, pour la préparation d'un agent pour le traitement de poissons stressés.

8. Utilisation des humates,selon la revendication 4, pour la détoxication de l'eau.

9. Utilisation des humates, selon la revendication 4, pour la détoxication de surfaces et d'objets solides.

10. Utilisation des humates, selon la revendication 4, pour la préparation d'un médicament nasal.

11. Utilisation des humates, selon la revendication 4, pour la préparation d'un agent antipelliculaire.
